# EUROPEAN PATENT SPECIFICATION

(11) **EP 0 502 416 B1**
(45) Date of publication and mention of the grant of the patent: **03.12.1997**
(21) Application number: 92103334.6
(22) Date of filing: 27.02.1992
(51) Int. Cl.: C07K 16/42, C07K 16/22, A61K 39/395

(54) **Stimulation of growth with anti-idiotypic antibodies raised against an antibody specific for porcine somatotropin**
Wachstumsstimulierung durch antiidiotypische Antikörper erzeugt gegen einen für Schweinewachstumshormon spezifischen Antikörper
Stimulation de la croissance par des anticorps anti-idiotypiques dirigés contre un anticorps contre somatotropine porcine

(30) Priority: 04.03.1991 US 680182
(43) Date of publication of application: 09.09.1992
(73) Proprietor: AMERICAN CYANAMID COMPANY, Wayne, NJ 07470-8426 (US)
(72) Inventor: Wang, Bosco S., Cranbury, NJ 08512 (US); Lumanglas, Araceli L., Jersey City, NJ 07375 (US); Szewcyzk, Eugene, Jersey City, NJ 07375 (US)
(74) Representative: Wächtershäuser, Günter, Prof. Dr.

(56) References cited:
- EP-A- 0 429 788
- EP-A- 0 439 797
- JOURNAL OF ENDOCRINOLOGY, vol. 125, no. 1, April 1990, London (GB); M. GARDNER et al., pp. 53-59/
- JOURNAL OF ENDOCRINOLOGY, vol. 11, Suppl., 1986, London (GB); C.A. MORRISON, no. 14/
- MOLECULAR & CELLULAR ENDOCRINOLOGY, vol. 62, no. 1, March 1989, Shannon (IE); G. LEFEVRE et al., pp. 125-133/
- CLINICAL IMMUNOLOGY & IMMUNOPATHOLOGY, vol. 21, 1981, New York, NY (US); A. NISONOFF et al., pp. 397-406/

## Description

### FIELD OF THE INVENTION

This invention relates to the generation of anti-idiotypic antibodies by administering to vertebrates an antibody to a growth potentiating polypeptide. Vertebrates so treated generate an anti-idiotypic antibody which mimics the activity of the polypeptide and stimulates growth in the vertebrates.

### BACKGROUND OF THE INVENTION

EP-A-439 797 discloses monoclonal antibodies which react with growth hormone binding protein. "J. Endocrinol., Vol. 125, p. 53-59 (1990)" and "J. Endocrinol., Vol. 114, Supplement No. 14 (1986)" disclose anti-idiotypic antibodies to growth hormones which function as hormone surrogates showing growth enhancing effects.

Various classes of polypeptides have been identified which potentiate growth of cells in vertebrates or which potentiate the growth of vertebrates per se. Such classes of polypeptides include the somatotropins, cytokines and lymphokines, insulin-like growth factors, transforming growth factors, and fibroblast growth factors.

In particular, somatotropin is a polypeptide secreted by the anterior pituitary in warm-blooded animals and acts through specific cell surface receptors located primarily in the liver (Hughes, J. P. and Friesen, H. G., Ann. Rev. Physiol., 47, 469-482 (1985)). Somatotropin is useful to enhance growth of such animals as bovine (cattle), porcine (pigs), caprine (goat), avian (chicken, turkey, geese, etc.) and rabbit, among others. Somatotropin is also useful to enhance the growth of other vertebrates, such as fish.

Specifically, the growth hormone porcine somatotropin (pST) is native to swine and accounts for maturation of the animal, including increasing the growth rate and the lean to fat ratio. It has been found that pST is a single chain polypeptide of 191 amino acids with two cystine bridges linking residues 53-164 and 181-189, respectively (Abdel-Meguid, S.S., et al., Proc. Nat. Acad. Sci., 84, 6434-6437 (1987).

Because endogenous amounts of pST are small, efforts have focused on the preparation of exogenous pST for use in large-scale agriculture. Efforts have also been directed to the identification of small portions of the pST molecule, the generation of antibodies to those portions, and the administration of those antibodies together with pST to enhance growth. See, for example, published European Patent Application 284,406. Administration of such growth promoting agents, either alone or in combination, must be frequently provided in order to promote the growth of the animal.

### SUMMARY OF THE INVENTION

Although the approaches described above have shown promise for enhancing growth of vertebrates, there is a continuing need to develop additional strategies and techniques in this area.

Accordingly, it is an object of this invention to stimulate growth by administering to vertebrates an antibody to a growth potentiating polypeptide, such that an anti-idiotypic antibody is generated which mimics the activity of the polypeptide.

It is a further object of this invention to stimulate growth by administering to vertebrates a fragment from an antibody to a growth potentiating polypeptide in order to generate an anti-idiotypic antibody which mimics the activity of the polypeptide.

These objects are accomplished in the description of the invention set forth below.

### BRIEF DESCRIPTION OF THE FIGURES

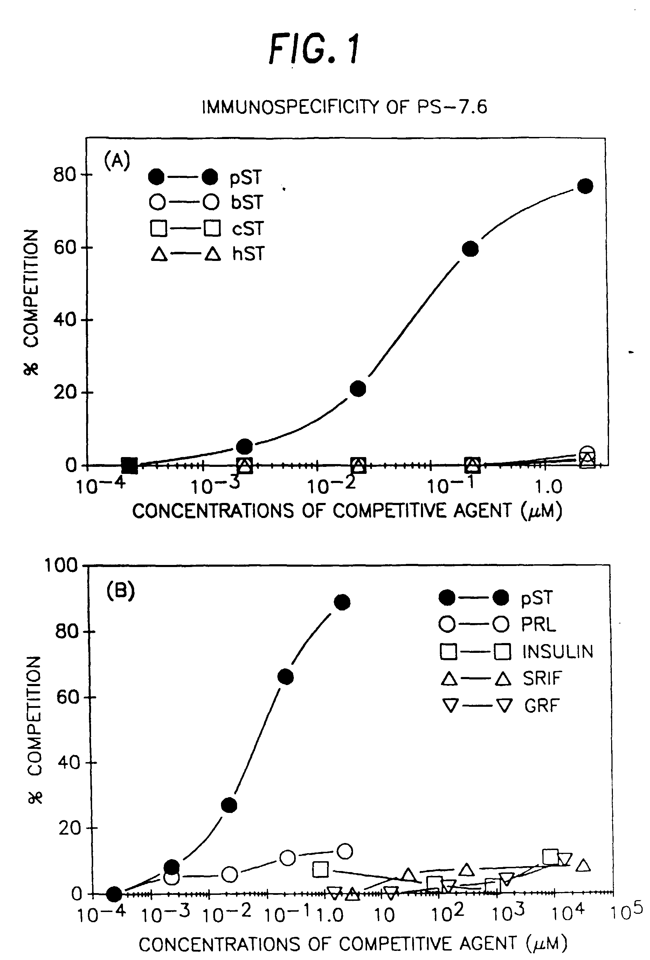

Figure 1, panel A, depicts a study of the immunospecificity of monoclonal antibody PS-7.6 to various antigens, where pST, bST, cST and hST are, respectively, porcine, bovine and chicken somatotropin, and hST is human growth hormone. The immunospecificity is demonstrated by a competition radioimmunoassay. Figure 1, panel B, depicts a similar competition radioimmunoassay, where PRL is prolactin, SRIF is somatostatin and GRF is growth hormone releasing factor.

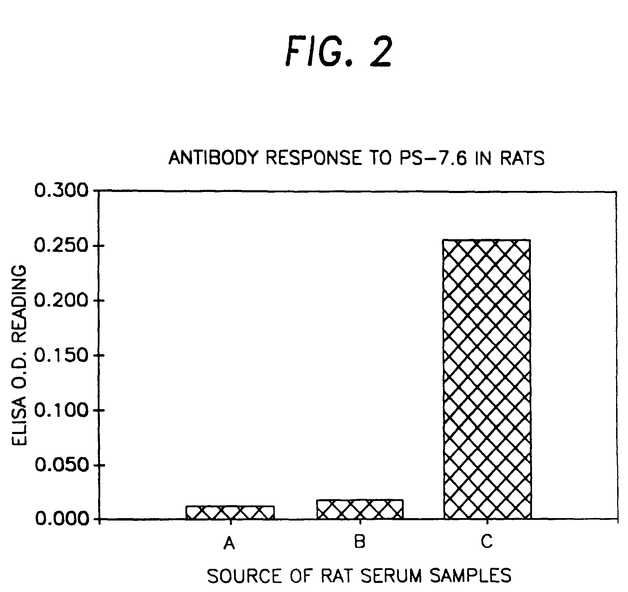

Figure 2 depicts a study wherein three groups of hypophysectomized (hypox) rats are (A) untreated, (B) treated with 5 µg, pST, or (c) treated with a combination of 5 µg, pST plus one mg monoclonal antibody PS-7.6. After two months, serum samples are harvested from each of the three groups of animals and tested for the presence of anti-monoclonal antibody PS-7.6 antibodies which bind to monoclonal antibody PS-7.6. ELISA plates are coated with monoclonal antibody PS-7.6 and serum samples from each of the three groups are then added. Goat anti-rat immunoglobulin (Ig) antibody conjugated with alkaline phosphatase is then added to the plates. Then a substrate, p-nitrophenyl phosphate, is added for color development and the resulting optical density is measured on a spectrophotometer.

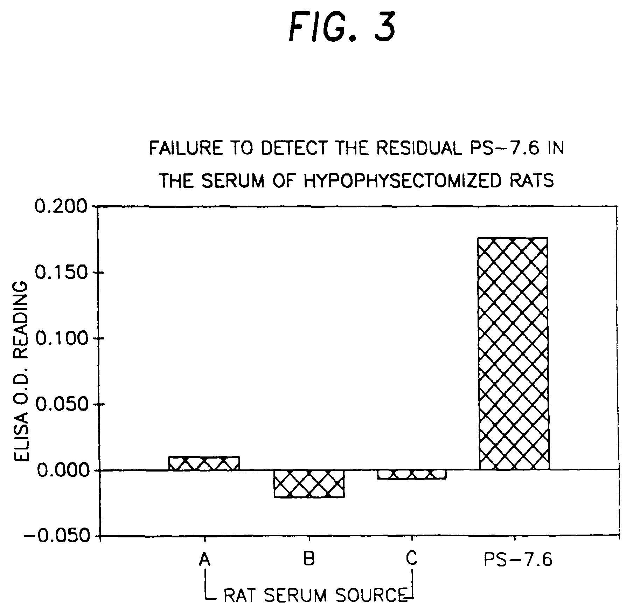

Figure 3 depicts a study wherein ELISA plates are coated with serum samples from each of the three groups of hypox rats described in Figure 2. Goat anti-mouse Ig conjugated with alkaline phosphatase is added to the plates, followed by the addition of the substrate. The samples are tested for the presence of residual monoclonal antibody PS-7.6. A fresh source of monoclonal antibody PS-7.6 is used as a positive control.

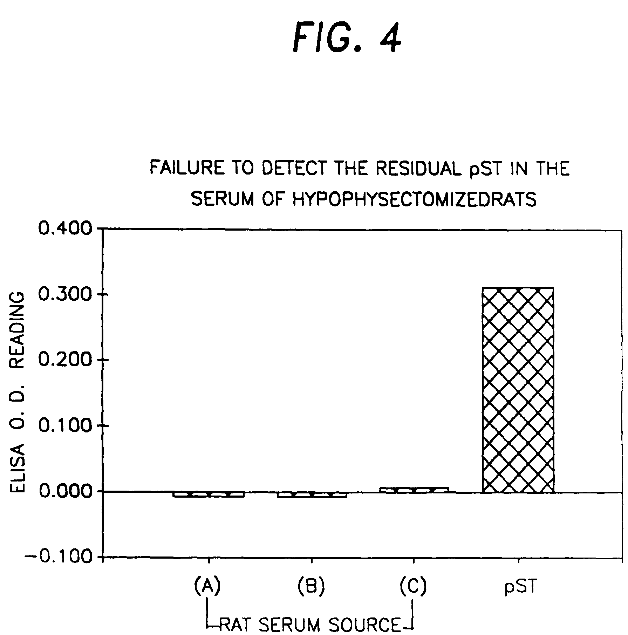

Figure 4 depicts a study similar to that of Figure 3, except that rabbit anti-pST polyclonal antibody is added to the plates, followed by the addition of goat anti-rabbit Ig antibody conjugated with alkaline phosphatase and then the substrate and the serum samples tested for the presence of residual pST. A fresh source of pST is used as a positive control.

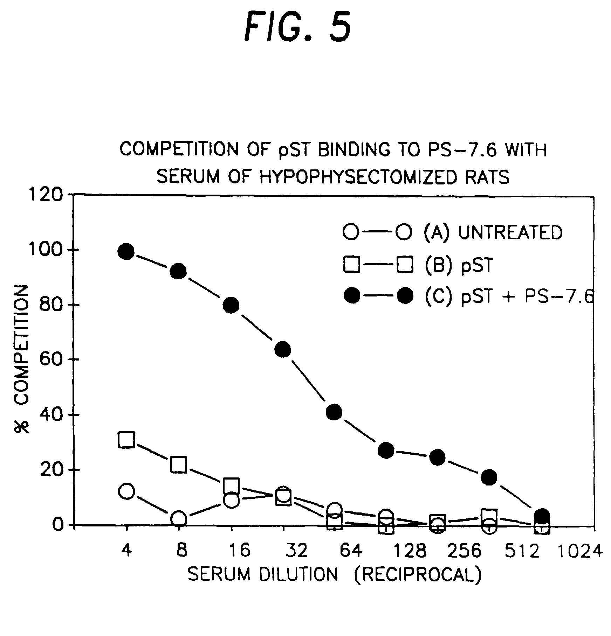

Figure 5 depicts a competition study wherein microtiter wells are coated with monoclonal antibody PS-7.6. Serum samples from the three groups of hypox rats described in Figure 2 are added to the wells at different dilutions together with ¹²⁵I-pST (50,000 - 80,000 cpm). After incubution, radioimmunoassay (RIA) plates are washed and individual wells are counted in a gamma counter.

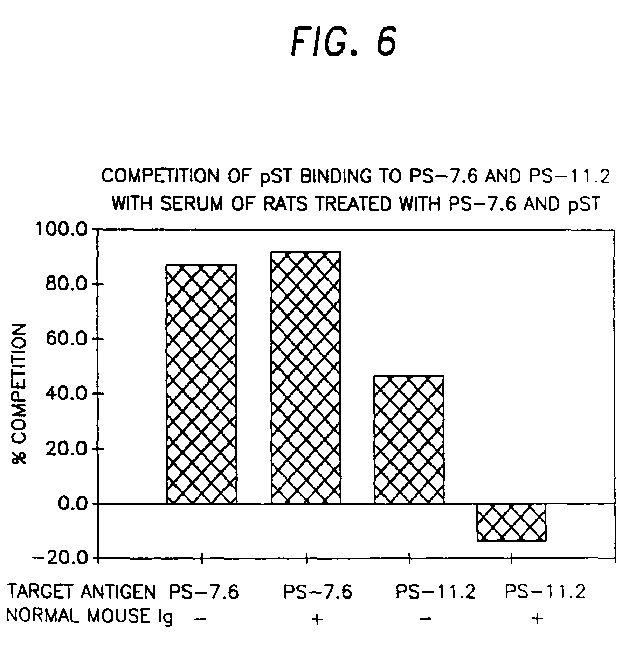

Figure 6 depicts a repetition of the competition study of Figure 5, except that only the serum from the group C rats (diluted to 5%) treated with both pST and monoclonal antibody PS-7.6 is used to examine the further addition of 1% normal mouse Ig (bar 2) as compared to the absence of normal mouse Ig (bar 1), as well as the further addition normal mouse Ig to wells coated with a different anti-pST monoclonal antibody PS-11.2 (bar 4), which is compared to the absence of normal mouse Ig (bar 3).

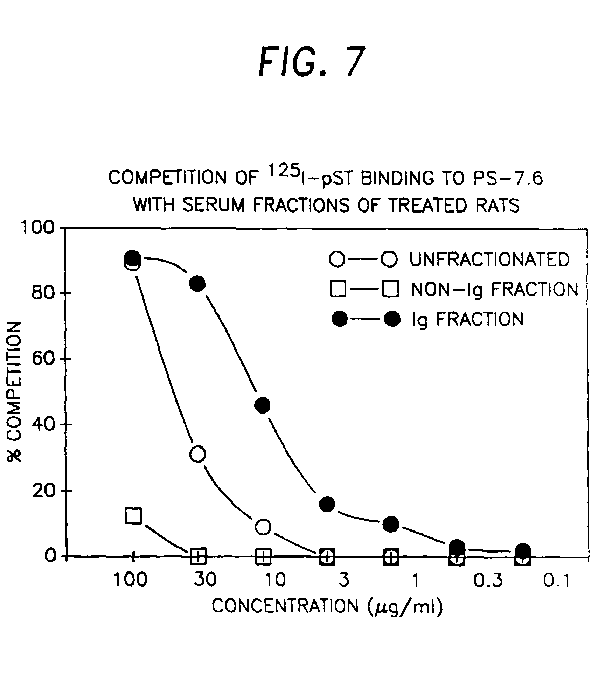

Figure 7 depicts a repetition of the competition study of Figure 5, except that the serum from the group C rats is compared with the bound (Ig) and unbound (non-Ig) fractions of that serum obtained by fractionation with Protein G affinity column chromatography.

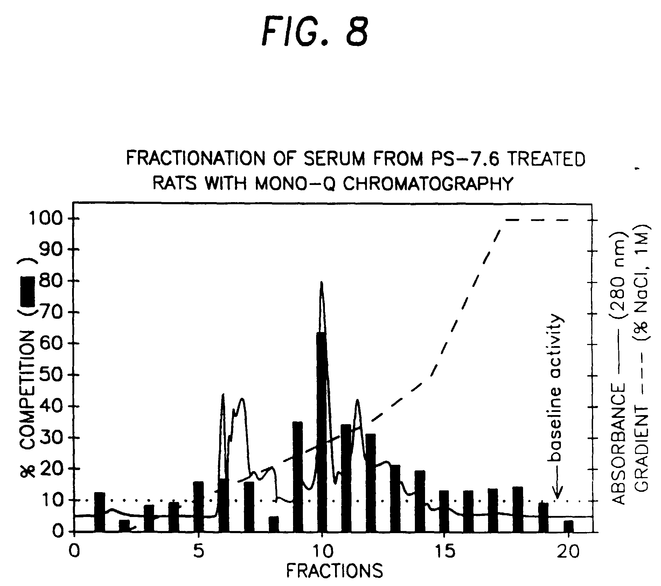

Figure 8 depicts a further chromatographic fractionation of the serum from the group C rats by FPLC-ion exchange chromatography. Fractions are then tested in a competition RIA for competition with ¹²⁵I-pST in binding to monoclonal antibody PS-7.6.

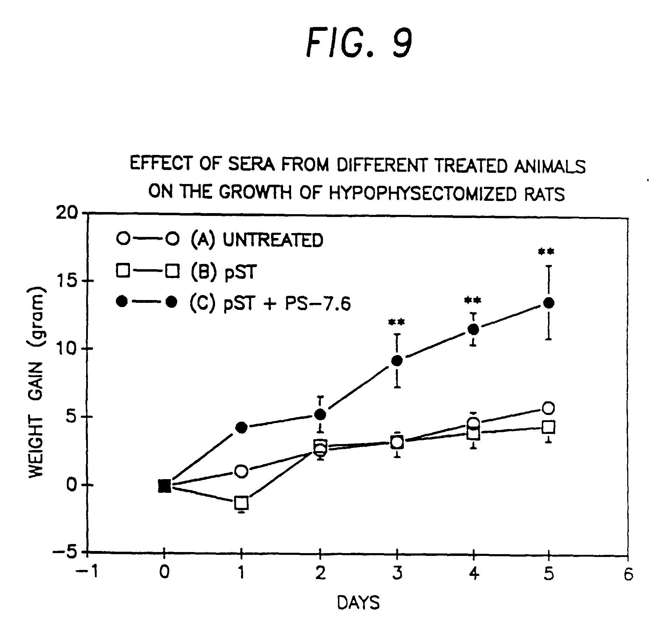

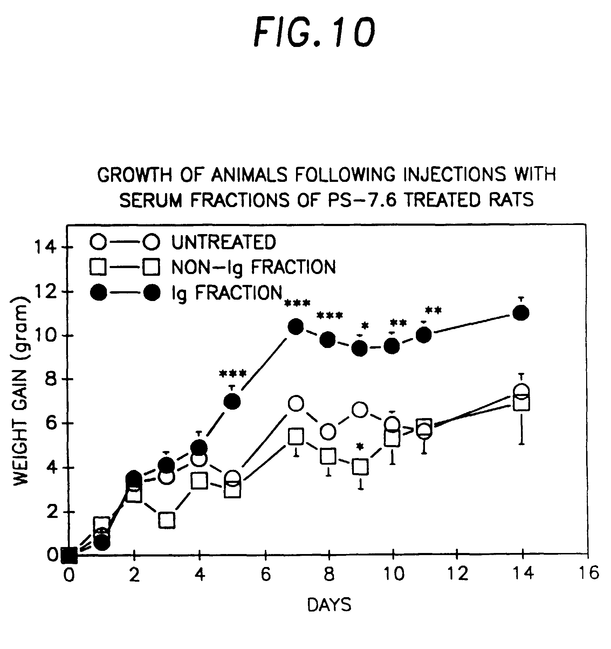

Figure 9 depicts the effect of the daily administration (for 5 days) of 0.5 ml of the three groups of sera upon the growth of a second set of hypox rats. The asterisks in this Figure, as well as Figure 10, represent statistically significant results as follows: * = p<0.05; ** = p<0.01; ***= p<0.001.

Figure 10 depicts the effect of the daily administration (for 5 days) of one mg per injection of the unfractionated and fractionated sera of group C (from Figure 7) upon the growth of a second set of hypox rats.

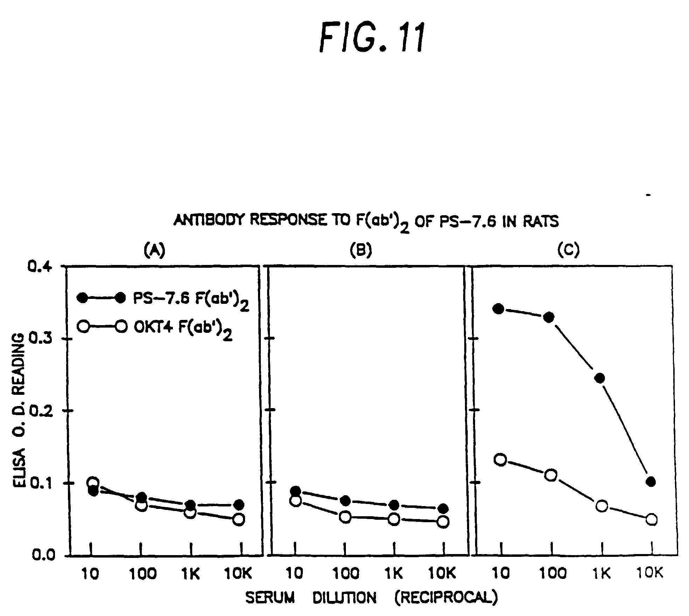

Figure 11 depicts a study wherein ELISA plates are coated with either an F(ab')₂ fragment of monoclonal antibody PS-7.6 or an F(ab')₂ fragment of monoclonal antibody OKT4 (as a control) and serum samples are assayed as in the study depicted in Figure 4. Panel A: Serum from untreated rats; Panel B: Serum from rats treated with pST; Panel C: Serum from rats treated with pST plus monoclonal antibody PS-7.6.

### DETAILED DESCRIPTION OF THE INVENTION

This invention is directed to the generation of anti-idiotypic antibody PS 7.6 produced by the hybridoma designated ATCC 10416 according to Claim 1, whereby the antibody is raised against an antibody to a growth potentiating polypeptide. An anti-idiotypic antibody resembles in pertinent part a corresponding antigen which induces the formation of an antibody. In particular, an anti-idiotypic antibody bears the internal image of a region of an antigen and thus mimics the shape of the antigen. In order for an anti-idiotypic antibody to mimic the desired activity of an antigen, the anti-idiotypic antibody must bind to in or near the same region of the receptor for an antigen as the antigen itself binds to. Stated another way, in order to generate an anti-idiotypic antibody which mimics the activity of the antigen, a monoclonal or polyclonal antibody to a corresponding antigen must bind to that antigen in or near the same region as the antigen binds to its receptor.

The antigen-mimicking anti-idiotypic antibodies of this invention are generated from antibodies to a wide variety of growth potentiating polypeptides. The polypeptides include the somatotropins, such as porcine, bovine, caprine, avian, rabbit and fish somatotropins. The polypeptides also include cytokines and lymphokines, such as the interleukins, the interferons or other cytokines or lymphokines which have immunomodulating activity. The polypeptides further include somatostatin and somatomedins, such as insulin-like growth factors. Additional polypeptides include insulin, prolactin, growth hormone releasing factor, transforming growth factors, fibroblast growth factors, epidermal growth factors and colony stimulating factors. The growth potentiating polypeptides include proteins, peptides and physiologically active portions thereof.

The growth promoting polypeptide exemplified herein is porcine somatotropins. However, the concept of generating mimicking anti-idiotypic antibodies may be applied to other growth potentiating polypeptides by a person skilled in the art utilizing the techniques described herein.

In one embodiment the anti-idiotypic antibodies may be generated in vivo by administering to a vertebrate an antibody to a somatotropin. In a variation of this embodiment, a somatotropin is administered to the vertebrate together with the antibody. The vertebrate then generates an anti-idiotypic antibody which mimics the effect of the somatotropin, such that the growth of the vertebrate is stimulated.

In another embodiment serum may be taken from a vertebrate which generates anti-idiotypic antibodies to a somatotropin and may then be administered to a second vertebrate. It is found that the second vertebrate also achieves a stimulation in weight gain, because of the presence of the anti-idiotypic antibodies originally present in the serum of the first vertebrate. In either of these embodiments, the first or second vertebrate may be a warm-blooded animal or a fish.

In both of these embodiments of this invention, the anti-idiotypic antibodies are generated from either monoclonal antibodies or polyclonal antibodies. After a description of how monoclonal antibodies to a somatotropin are generated is first presented, these embodiments will be described with respect to monoclonal antibodies, followed by a description with respect to polyclonal antibodies.

Monoclonal antibodies are prepared by immunizing mice with porcine somatotropin, removing the spleens of the mice, preparing suspensions of lymphocytes, fusing these lymphocytes to mouse myeloma cells, culturing the cells and collecting supernatants of surviving hybridomas for antibody screening by solid-phase enzyme-linked immunosorbent assay (ELISA). Those hybridomas which produce desired antibodies are further subcloned and injected in mice. Ascites are then collected from the peritoneal cavities of mice and immunoglobulin (Ig) is purified by either ammonium sulfate precipitation or a protein A affinity column by fast protein liquid chromatography (FPLC). Samples of Ig so purified are assayed against antigens using ELISA to identify the antibodies formed.

Although the somatotropin exemplified in this application is pST, a person skilled in the art may utilize the techniques described herein to generate monoclonal antibodies to other somatotropins. The pST used may be isolated from natural sources or may be prepared using recombinant techniques such as those described in published European patent applications 104,920 or 111,389. The sources of and the method of isolation/preparation of pST itself forms no part of this invention. The antibodies may also be used together with recombinant pST in which the amino acid sequence of native pST has been modified using a technique such as site-directed mutagenesis, so long as the growth enhancing function of pST is maintained. See, for example, published European patent application 355,460; see also published European patent application 303,972.

Monoclonal antibodies designated PS-3.12, PS-7.6 and PS-8.3 are preferred among the monoclonal antibodies so generated. Monoclonal antibody PS-7.6 is not within the scope of the invention, but is the only primary antibody employed in the invention. This antibody recognizes determinants in or near the binding region of pST for its receptor.

Monoclonal antibody PS-7.6 is an IgG₁ subtype immunoglobulin with k light chain characteristics. An immunospecificity study (Figure 1) indicates that it is highly reactive with pST, but not with bST, cST, PRL, SRIF or GRF.

A monoclonal antibody so generated is administered to a vertebrate, either alone or together with a somatotropin. For the purpose of exemplification, the vertebrates used are hypophysectomized (hypox) rats. Hypox-rats are growth-deficient as a result of surgical removal of their pituitary glands. Hypox-rats serve as a useful model for studying the effect of somatotropin on growth (Groesbeck, M. D. and Parlow, A. F., Endocrinology, 120, 2582-2590 (1987). After a sufficient period of time for anti-idiotypic anitbodies to be generated, serum samples are harvested from the hypox rats and analyzed.

In order to ascertain the nature of the serum samples, a series of experiments is conducted. First, ELISA plates are coated with the monoclonal antibody and the serum samples are added. For comparison, samples of serum from hypox rats treated with somatotropin alone and from untreated rats are also added. Next, goat anti-rat Ig antibody conjugated with alkaline phosphatase is added to the plates and incubated, followed by the addition of a substrate such as p-nitrophenyl phosphate for color development. Optical density readings indicate that only the serum from the rats treated with the somatotropin and the monoclonal antibody contains an anti-mouse monoclonal antibody (Figure 2). Further experiments described below show this to be an anti-idiotypic antibody.

The next experiments demonstrate that the serum containing the anti-mouse antibody no longer contains the mouse monoclonal antibody or somatotropin. In one experiment, serum samples are used to coat ELISA plates. A fresh sample of mouse monoclonal antibody, not from serum, is used as a positive control. Further additions to the plates (goat anti-mouse Ig antibody conjugated with alkaline phosphatase, followed by a substrate) are made. Optical density readings indicate that no statistically significant levels of the mouse monoclonal antibody are present in the serum (Figure 3).

In another similar experiment, serum samples are used to coat ELISA plates. A fresh sample of somatotropin, not from serum, is used as a positive control. Further additions to the plates (rabbit anti-somatotropin antibody, followed by goat anti-rabbit Ig antibody conjugated with alkaline phosphatase, then a substrate) are made. Optical density reading indicate that no statistically significant levels of pST are present in the serum (Figure 4).

Competition assays establish that the serum containing the anti-mouse monoclonal antibody competes with somatotropin for binding to a monoclonal antibody to somatotropin. Radiolabelled somatotropin binds to the monoclonal antibody. Serum is added at increasing dilutions. It is found that, at higher serum concentrations, the counts of radiolabelled somatotropin are reduced, indicating greater competition from the serum (Figure 5). In turn, when normal Ig is added, the competition from the serum is not reduced (Figure 6, bars 1 and 2). This indicates that the serum competes with the somatotropin for binding at the variable, rather than the constant region of the monoclonal antibody.

The preceding experiment is repeated using a different monoclonal antibody (PS-11.2). It is found that the serum competes with the somatotropin to a lesser degree (Figure 6, bars 3 and 4), and this competition is targeted at the constant region, because normal mouse Ig totally abrogates this effect. This indicates that different monoclonal antibodies recognize different epitopes of the somatotropin. In turn, the serum must contain antibodies which share an epitope with the somatotropin which is recognizable by only one of the monoclonal antibodies (PS-7.6).

Fractionation of the serum by techniques such as affinity column chromatography yields Ig and non-Ig fractions. Comparison of the two fractions with unfractionated serum in the competition assay results in greatest competition with the somatotropin for binding to the monoclonal antibody being found in the Ig fraction (Figure 7). Fractionation of the serum by FPLC-ion exchange chromatography, followed by testing of fractions in a competition assay, serves to isolate the most active moiety (Figure 8). Analysis of the most active fraction (fraction 10) by SDS-PAGE confirms that it contains Ig.

When the results of the assays depicted in Figure 2-8 are analyzed, the conclusion is reached that the serum contains anti-idiotypic antibodies which bind to monoclonal antibodies to a somatotropin and share an epitope with somatotropin. Because of the structural similarity with an epitopic region of the somatotropin molecule, these particular anti-idiotypic antibodies are able to mimic the activity of the somatotropin, that is, to stimulate growth in the vertebrate. Anti-idiotypic antibodies may be generated which lack this structural similarity. Such antibodies will not mimic the activity of the somatotropin and do not constitute part of this invention.

The growth stimulating effect of the anti-idiotypic antibodies is confirmed by administering serum into a second set of animals. Animals receiving the serum have a statistically significant weight gain (Figure 9). Thus, the serum contains anti-idiotypic antibodies which mimic the growth stimulating effect of a somatotropin.

When the serum is fractionated by affinity chromatography and the Ig and non-Ig fractions are administered to a different set of animals, the animals receiving the Ig fraction have a statistically significant weight gain, in contrast to animals receiving the non-Ig fraction (Figure 10). This further confirms that the anti-idiotypic antibodies are found in the Ig fraction.

As mentioned previously, the anti-idiotypic antibodies are also generated from polyclonal antibodies. Polyclonal antibodies to a somatotropin are generated and purified from immunized warm-blooded animals such as swine and rabbits. Polyclonal antibodies are generated by immunizing vertebrates with such a somatotropin.

Polyclonal antibodies are recovered by first obtaining a blood sample from an immunized animal after a time sufficient from administration of the somatotropin for antibodies to be formed. The serum (which contains the antibodies) is isolated by conventional means such as centrifugation. Serum is separated into fractions containing Ig and lacking immunoglobulin (non-Ig) by means such as affinity column purification. Only the Ig fraction contains antibodies to the somatatropin, along with other antibodies.

The polyclonal antibodies specific for the somatotropin are next separated from the other antibodies by passing the Ig fraction through an affinity column to which somatotropin has been bound. Only those antibodies which are specific for the somatotropin will bind to the column. The desired antibodies are then eluted from the column by reducing the pH of the buffer solution passed through the column. The antibodies are then isolated from the eluate by sodium dodecyl sulfate-polyacrylamide gel electrophoresis (SDS-PAGE). The purity of the antibodies so isolated is greater than 98% as determined by SDS-PAGE. The antibody titer level is assayed using ELISA according to conventional procedures.

In order to select polyclonal antibodies which inhibit binding of the somatotropin to its receptor (and thus can generate mimicking anti-idiotypic antibodies), the following procedure is used. A somatotropin receptor is covalently linked to its corresponding somatotropin and, in turn, is bound to a protein affinity column. Antisera containing polyclonal antibodies is then passed through the column. Polyclonal antibodies which bind to the column recognize the somatotropin in regions not involved in binding to the receptor. Such polyclonal antibodies are not appropriate for generating mimicking anti-idiotypic antibodies.

Antisera which passes through the column without binding contains polyclonal antibodies which would have bound to the somatotropin in or near the receptor binding region, but for the fact that the somatotropin was already bound to the column on the receptor. Such eluted polyclonal antibodies are then administered to a vertebrate in the same fashion as monoclonal antibodies to generate mimicking anti-idiotypic antibodies.

Specifically with regard to the somatotropins, the anti-idiotypic antibodies mimic a second growth-related function of the somatotropin in addition to stimulating growth per se. It is known that somatotropins such as pST exert a repartitioning effect upon the vertebrate; pST increases the proportion of lean and decreases the proportion of fat for a given weight of the vertebrate. The anti-idiotypic antibodies also exert this repartitioning effect.

The anti-idiotypic antibodies of this invention may be administered by conventional means such as subcutaneous injection, intramuscular injection and intravenous flow, as well as transdermal and oral administration. It is preferred to administer these antibodies in association with a pharmaceutically acceptable carrier, diluent or adjuvant known in the art. The dosage to be administered and the number of doses is determined by means known to those skilled in the art.

As described above, the anti-idiotypic antibodies compete with the somatotropin for binding at the variable, rather than the constant region of the monoclonal antibody. An additional embodiment of this invention utilizes fragments of the monoclonal antibody or the polyclonal antibody which contain, among other things, the variable region of the antibody. The fragments are used to generate anti-idiotypic antibodies which are used to stimulate growth of vertebrates. The fragments must contain a region of the antibody which participates in binding with the receptor.

An antibody treated with a digestive enzyme such as pepsin yields F(ab')₂ fragments. These fragments contain the light chain and a portion of the heavy chain of the antibody, as well as an antigenic binding site and the variable region of the antibody, where the amino acid sequence differs depending on the source of the antibody. Similarly, an Fᵥ fragment is prepared. An Fᵥ fragment consists of variable portions of one heavy and one light chain of the antibody. Other appropriate fragments are generated which contain a region which participates in binding with the receptor. In addition to enzymatic cleavage, the fragments are obtained by ascertaining the amino acid sequence of the fragment, followed by chemical synthesis using conventional techniques.

Alternatively, the fragments are obtained by recombinant DNA technology using conventional techniques. DNA is extracted from the hybridoma cell lines which produce the monoclonal antibodies of interest. The DNA sequence is mapped and the sequence coding for the variable region of the monoclonal antibody is identified. A technique such as polymerase chain reaction (PCR) is used to amplify the DNA sequence coding for the variable region. The products of PCR amplification are electrophoresed on an agarose gel and analyzed. The DNA sequence coding for the desired fragment is then inserted into a host cell with an appropriate expression vector, and the fragment expressed, isolated and purified.

Such fragments behave in a manner similar to the entire monoclonal antibody (or polyclonal antibody). Fragments are coated onto ELISA plates and serum containing anti-idiotypic antibodies. The serum recognizes the fragments from the entire monoclonal antibody, but not fragments from an unrelated monoclonal antibody (Figure 11). Thus, fragments of monoclonal antibodies or polyclonal antibodies of interest are administered to vertebrates to generate anti-idiotypic antibodies which stimulate growth in the same manner as anti-idiotypic antibodies generated from the entire monoclonal antibody or polyclonal antibody.

In yet another embodiment the anti-idiotypic antibodies are obtained using hybridoma technology in a manner analogous to that used to obtain monoclonal antibodies. Mice are immunized with a monoclonal antibody (alone or together with a growth potentiating polypeptide), the spleens of the mice are removed, suspensions of lymphocytes are prepared, the lymphocytes are then fused to mouse myeloma cells, the cells are cultured, and supernatants of surviving hybridomas are collected and screened for antibodies by ELISA. Those hybridomas which produce desired antibodies are further subcloned and injected in mice. Ascites are then collected from the peritoneal cavities of mice and Ig is purified by either ammonium sulfate precipitation or a protein A affinity column by FPLC. Samples of Ig so purifed are assayed against antigens using ELISA to identify the anti-idiotypic antibodies formed.

### EXAMPLE 1

### 1. Generation of Monoclonal Antibodies, Including Monoclonal Antibody PS-7.6

Balb/C mice, six to ten weeks of age, are purchased from Charles River Breeding Laboratories, Wilmington, MA. These mice are immunized with 100µg pST emulsified in complete Freund's adjuvant and boosted with 50µg of pST every three weeks thereafter. Their spleens are removed three days after the last boosting and single cell suspensions of lymphocytes are prepared. These lymphocytes are fused with SP2/0 mouse myeloma cells lacking hypoxanthine phyosphoribosyl transferase (HPRT) with 50% polyethylene glycol, suspended in Dulbecco's modified Eagle medium (D-MEM) containing 20% fetal calf serum (Gibco), 0.175µg/ml aminopterin, 13.6µg/ml hypoxanthine, 3.88µg/ml thymidine and 50µg/ml gentamicin (HAT medium), and finally dispensed in 96-well culture plates. After being cultured for 10-14 days, supernatants of the hybridomas which survive due to the HPRT-positive phenotype of the lymphocytes (approximately 30%) are collected for antibody screening in a solid-phase ELISA. Those being determined to produce appropriate antibodies (by consistently achieving high titers in supernatants -- approximately 25% of the approximately 30% referred to above, for a net of approximately 7%) are further subcloned by a limited dilution procedure to produce antibodies of interest.

Three hybridomas produce clones of particular interest. Samples of these hybridomas have been deposited on April 12, 1990 with the American Type Culture Collection, 12301 Parklawn Drive, Rockville, MD 20852, and have been assigned accession numbers as shown in Table 1, together with the designations of the monoclonal antibodies (abbreviated as "MoAb") of interest produced by those hybridomas:

**TABLE 1**

| Hybridoma | Accession Number | Monoclonal Antibody |
|---|---|---|
| PS-3.12 | ATCC HB 10415 | MoAb PS-3.12 |
| PS-7.6 | ATCC HB 10416 | MoAb PS-7.6 |
| PS-8.3 | ATCC HB 10417 | MoAb PS-8.3 |

The selected clones are injected intraperitoneally into Balb/C mice which are primed with pristane for the production of antibody-containing ascites. Identification of the isotype of the monoclonal antibodies is carried out with an antibody isotyping kit (Zymed Laboratories, South San Francisco, CA). The monoclonal antibody designated PS-7.6 is selected as exemplary for further study in this Example 1.

### 2. Preparation of Antibody

Ascites are collected from the peritoneal cavities of mice and Ig is purified with a 50% ammonium sulfate precipitation technique. Alternatively, samples are diluted to 50% with binding buffer (3M NaCl, 1.5M glycine, pH 8.9) and loaded onto a preparative Protein A Superose® HR 16/5 column on a FPLC system (Pharmacia, Piscataway, NJ). The non-Ig fraction is eluted from the column with the binding buffer and the bound Ig is subsequently collected by rinsing the column with 0.1M citric acid, pH 3. It is immediately neutralized to pH 7-8 with 2M Tris buffer, pH 8.2. Antibody prepared by both procedures is extensively dialyzed against phosphate-buffered saline (PBS), concentrated by ultrafiltration (Amicon, Danvers, MA), aliquoted, and finally stored at -20°C until use.

### 3. Solid Phase ELISA

Antigens are dissolved in PBS and 1µg in 100µl is added to each well of a 96-well flat bottom polystyrene plate. After being incubated for one hour, the plate is washed three times with PBS containing 0.05% Tween®-20 by an automatic plate washer (Dynatech Wash II, Chantilly, VA). Each well is dispensed with 200µl of 2% bovine serum albumin (Sigma) and the plate is incubated for another hour. Test samples are added to the wells, incubated for 30 minutes, washed six times with PBS, and added with 100µl of alkaline phosphatase-conjugated goat anti-mouse IgG F(ab')₂ (Zymed Laboratories, South San Francisco, CA). The plate is washed again after a 30 minute incubation and 100µl of p-nitrophenyl phosphate (1mg/ml, Sigma) in 0.1M diethanolamine, pH 10.3, is added as substrate for color development. Finally, the colorimetric response is recorded as optical density (OD) by an ELISA plate reader at a wavelength of 405 nm. The incubation procedure is always performed at 37°C.

### 4. Characterization of Monoclonal Antibody PS-7.6

The immunospecificity of the PS-7.6 monoclonal antibody is tested in a solid phase ELISA. Various antigens are coated in the wells of a 96-well plate and a series of dilutions of monoclonal antibody PS-7.6 ascites is added for interaction. Figure 1 indicates that monoclonal antibody PS-7.6 responds to pST antigen markedly well, but not with bST, cST, PRL, SRIF or GRF.

The isotype of monoclonal antibody PS-7.6 determined by an antigen-dependent, biotin-avidin amplified ELISA is IgG₁ with k light chain characteristics.

### EXAMPLE 2

Steps 1-4 of Example 1 are repeated to generate, purify and characterize monoclonal antibodies PS-3.12 and PS-8.3.

### EXAMPLE 3

### 1. Generation of Anti-Idiotypic Antibodies

Hypox rats are treated with a combination of 5µg pST plus one mg monoclonal antibody PS-7.6. After two months, serum samples are harvested and analyzed. As described below, the serum contains anti-idiotypic antibodies to monoclonal antibody PS-7.6.

### 2. Presence of Anti-monoclonal antibody PS-7.6 Antibodies in Serum

An experiment is conducted to ascertain whether the sera of the hypox rats of part 1 of Example 3 contain a component which binds monoclonal antibody PS-7.6. Serum samples are taken from the hypox rats two months after injection with pST and monoclonal antibody PS-7.6. For comparison, serum samples are also taken from hypox rats two months after treatment with 5µg pST alone and from untreated rats. ELISA plates are coated with monoclonal antibody PS-7.6 and serum samples from each of the three groups are then added. Goat anti-rat Ig antibody conjugated with alkaline phosphatase is then added to each plate and incubated at 37°C for 30 minutes. Then a substrate, p-nitrophenyl phosphate is added for color development and the resulting optical density is measured on a spectrophotometer. The results, as depicted in Figure 2, indicate that only the serum from the rats originally treated with pST and monoclonal antibody PS-7.6 contains an anti-monoclonal antibody PS-7.6-antibody. As shown below, this component is an anti-idiotypic antibody.

### 3. The Serum Which Generates the Anti-Idiotypic Antibodies Does Not Contain Residual Monoclonal Antibody PS-7.6 or pST

Two experiments are conducted to ascertain whether the sera of the hypox rats of part 1 of Example 3 continue to contain monoclonal antibody PS-7.6 or pST two months after injection of the hypox rats with those materials. In the first experiment, serum samples from the hypox rats are used as antigens to coat ELISA plates. For comparison, serum samples are also taken from hypox rats two months after treatment with pST alone and from untreated rats; both serum samples are also coated onto ELISA plates. A fresh sample of monoclonal antibody PS-7.6 in solution, not contained in serum, is used as a positive control. Goat anti-mouse Ig antibody conjugated with alkaline phosphatase is then added to the plates. Then a substrate, p-nitrophenyl phosphate is added for color development and the resulting optical density is measured on a spectrophotometer. The results, as depicted in Figure 3, indicate that no statistically significant levels of monoclonal antibody PS-7.6 are present in any of the serum samples. In contrast, the control plate coated with the fresh monoclonal antibody PS-7.6 evidences a statistically significant level of monoclonal antibody PS-7.6.

In the second experiment, the same three types of serum samples are used to coat ELISA plates. A fresh sample of pST in solution, not contained in serum, is used as a positive control. Rabbit anti-pST antibody is then added to the plates, followed by goat anti-rabbit Ig antibody conjugated with alkaline phosphatase and then the substrate as in the first experiment. The results, as depicted in Figure 4, indicate that no statistically significant levels of pST are present in any of the serum samples. In contrast, the control plate coated with the fresh pST shows a signficiant positive response.

### 4. Competition Assays of pST Binding to Monoclonal Antibodies with Sera

Microtiter wells of Immunlon-2 strips are coated with monoclonal antibody PS-7.6. In a solid phase radioimmunoassay (RIA), radiolabelled ¹²⁵I-pST (50,000-80,000 cpm) binds to the monoclonal antibody.

In a first competition assay, serum samples from the three sets of rats described previously are added to the wells at increasing two-fold dilutions. After a one hour incubation, RIA plates are washed and individual wells are counted in a gamma counter. The lower the counts, the greater the competition, as the unlabelled sera rather than the labelled pST binds to the monoclonal antibody.

Figure 5 depicts the results of the first competition assay. The serum from the rats immunized two months previously with both pST and monoclonal antibody PS-7.6 compete with ¹²⁵I-pST for the binding to monoclonal antibody PS-7.6, whereas sera from the other two groups had no effect.

In a second competition assay, the serum which competed in the first assay is used to examine the further addition of normal mouse Ig (bar 2 in Figure 6) as compared to the absence of normal mouse Ig (bar 1). The inhibition of the binding of ¹²⁵I-pST is not reduced in the presence of normal mouse Ig. This indicates that the serum competes with pST for binding at the variable, rather than the constant region of monoclonal antibody PS-7.6.

In a variation of this second competition assay, the strips are coated with another anti-pST monoclonal antibody, designated PS-11.2, and the absence (bar 3 of Figure 6) or presence (bar 4) of normal mouse Ig is tested. The serum competes with pST for binding to monoclonal antibody PS-11.2 at a lesser degree (approximately one-half) than binding to monoclonal antibody PS-7.6. This indicates that the monoclonal antibodies recognize different epitopes of pST and that the anti-idiotypic antibodies in the serum share an epitope with pST which is recognizable by monoclonal antibody PS-7.6, but not by monoclonal antibody PS-11.2. The inhibition of the binding of pST to the monoclonal antibody PS-11.2 by the serum is due solely to the steric hinderance of the anti-constant region of the mouse Ig, because the inhibitory effect is completely abolished by the presence of normal mouse Ig (bar 4).

### 5. The Effect of Fractionation of Serum Upon Competitive Binding

Serum from the rats immunized two months previously with both pST and monoclonal antibody PS-7.6 is fractionated by Protein G affinity column (Pharmacia, Piscataway, N.J.) chromatography. The sample is diluted with 3M NaCl and 1.5M glycine, pH 8.9, and loaded onto the column. The column is washed with 3M NaCl and 1.5M glycine, pH 8.9, and the fractions monitored by spectrophotometry at OD₂₈₀. The eluate contains the non-Ig fraction which does not bind to the column. The bound Ig fraction is released by washing the column with 0.1M citric acid and NaOH, pH 3.0, followed by neutralization with 3M Tris buffer, pH 7.5. The fractions are monitored as just described.

The non-Ig and Ig fractions are compared with unfractionated serum in the competition assay described previously. As depicted in Figure 7, competition with ¹²⁵I-pST for binding to monoclonal antibody PS-7.6 is greatest in the Ig fraction. This result indicates that the component which competes with pST is present mainly in the Ig fraction, which indicates that the component is anti-idiotypic antibodies.

### 6. FPLC-Ion Exchange Chromatography of Serum

The serum of the rats immunized two months previously with both pST and monoclonal antibody PS-7.6 is equilibrated with 20mM Tris buffer, pH 8, and subjected to FPLC-ion exchange chromatography using a Mono-Q® HR 5/5 column (Pharmacia, Piscataway, N.J.). Fractions are collected by eluting with a salt gradient (0-100% 1M NaCl), followed by de-salting with Centricon®-10 filters. The fractions are then tested for the ability to compete with ¹²⁵I-pST for binding to monoclonal antibody PS-7.6 in the RIA described previously.

The results depicted in Figure 8 indicate that the most active moiety in competing with ¹²⁵I-pST resides in fraction 10. When fraction 10 is subjected to SDS-PAGE and analyzed, it is confirmed that fraction 10 contains rat Ig and thus anti-idiotypic antibodies.

### 7. Effect of Sera Upon Growth of a Second Set of Animals

An experiment is conducted to ascertain whether the sera from the three groups of rats treated two months perviously contain a growth potentiating factor when injected into a second set of animals. Each serum is injected into one of three groups of a second set of hypox rats, eight rats per group. The rats receive a daily intraperitoneal injection of 0.5 ml of serum for five days. The weight gain of the rats is depicted in Figure 9.

The rats receiving serum from the rats previously immunized with both pST and monoclonal antibody PS-7.6 have a statistically significant weight gain. This indicates that the serum contains a factor which mimics the growth potentiating effect of pST. In contrast, serum from untreated rats or rats treated with pST alone had no statistically significant effect on the weight gain of rats receiving either serum.

### 8. Effect of Fractionated Serum Upon Growth of a Second Set of Animals

Serum from the animals which exhibit a growth potentiating effect as depicted in Figure 9 are subjected to fractionation by a Protein G affinity column according to the procedure described in part 6 of this Example 3. The non-Ig and Ig fractions, as well as untreated serum as a control, are injected into a second set of hypox rats, eight rats per group. The rats receive a daily injection (for the five days) of one mg in PBS per injection.

As indicated in Figure 10, the second set of rats which receive the Ig fraction demonstrate a statistically significant increase in weight gain. In contrast, rats which receive the non-Ig fraction or the untreated serum, do not demonstrate a statistically significant increase in weight gain. This experiments provides additional evidence that anti-idiotypic antibodies which mimic pST are found in the Ig fraction of serum of rats immunized with pST plus monoclonal antibody PS-7.6.

### 9. Antibody Response to an F(ab')₂ Fragment of Monoclonal Antibody PS-7.6

Monoclonal antibody PS-7.6 is digested for 12 hours with pepsin at 37°C in acetate buffer, pH 4.5, to generate F(ab')₂ fragments. Yields of the fragments are improved if the digestion is run for 15-18 hours. The digestion mixture is then diluted with phosphate-buffered saline (PBS), dialyzed first against PBS and then against distilled water, followed by lyophilization. These steps minimize the formation of heavy precipitates (which reduces yields) during the neutralization with buffer to pH 7.0.

The lyophilized material is then chromatographed using a Sephacryl® S200 column (Pharmacia, Piscataway, N.J.) and eluted with PBS. The fractions are monitored spectrophotometrically (O.D. 280). The fraction containing the F(ab')₂ fragment is subjected to SDS-PAGE and is found to have a molecular weight of approximately 100,000 daltons.

One set of ELISA plates is coated with the F(ab)'₂ fragments from monoclonal antibody PS-7.6. Another set of plates is coated with F(ab')₂ fragments from OKT4 mouse antibody (a monoclonal antibody to T lymphocytes; Ortho Pharmaceutical, Raritan, N.J.), which are obtained using the procedure described above. Serum samples from the three sets of rats are tested as described in part 2 at four different dilutions, each an order of magnitude more dilute than the preceding concentration.

The results are depicted in Figure 11. The serum from the rats immunized two months previously with both pST and monoclonal antibody PS-7.6 now contain anti-idiotypic antibodies which recognize the F(ab')₂ fragments of monoclonal antibody PS-7.6, but not the F(ab')₂ fragments of OKT4 mouse antibody (Panel C). Sera from untreated rats (Panel A) and rats previously treated with pST alone (Panel B) are ineffective.

## Claims (Claims for the following Contracting State(s): AT, BE, CH, LI, DE, DK, FR, GB, IT, LU, NL, PT, SE)

1. An anti-idiotypic monoclonal antibody, wherein said anti-idiotypic antibody is raised against the antibody designated PS-7.6 specific for porcine somatotropin, and obtainable from hybridoma ATCC 10416, wherein the anti-idiotypic antibody mimics the activity of porcine somatotropin and stimulates growth in a vertebrate.

2. A composition comprising:
(a) an effective growth stimulating amount of the anti-idiotypic monoclonal antibody of claim 1; and
(b) a pharmaceutically acceptable diluent or carrier.

3. A method for stimulating the growth of a vertebrate with the exception of humans, which comprises administering to the vertebrate an effective growth stimulating amount of the anti-idiotypic monoclonal antibody of claim 1.

## Claims (Claims for the following Contracting State(s): ES, GR)

1. A method for stimulating the growth of a vertebrate with the exception of humans, which comprises administering to a vertebrate an effective growth stimulating amount of an anti-idiotypic monoclonal antibody wherein said anti-idiotypic antibody is raised against the antibody designated PS-7.6 specific for porcine somatotropin, and obtainable from hybridoma ATCC 10416, wherein the anti-idiotypic antibody mimics the activity of porcine somatotropin and stimulates growth in the vertebrate.

2. A method for producing a composition for stimulating growth of a vertebrate which comprises formulating
(a) an effective growth stimulating amount of an anti-idiotypic monoclonal antibody wherein said anti-idiotypic antibody is raised against the antibody designated PS-7.6 specific for porcine somatotropin, and obtainable from hybridoma ATCC 10416, wherein the anti-idiotypic antibody mimics the activity of porcine somatotropin and stimulates growth in a vertebrate; and
(b) a pharmaceutically acceptable diluent or carrier.

3. A method for producing an anti-idiotypic monoclonal antibody mimicking the activity of porcine somatotropin and stimulating growth in a vertebrate with the exception of humans, said method comprising the step of raising an antibody against an antibody designated PS-7.6 which is specific for porcine somatotropin and which is obtainable from hybridoma ATCC 10416.

4. Use of an anti-idiotypic monoclonal antibody, obtainable according to the method of claim 3 for stimulating growth of a vertebrate, with the exception of humans.

5. Use of an anti-idiotypic monoclonal antibody, obtainable according to the method of claim 3 for producing a composition for stimulating growth of a vertebrate.

## Patentansprüche (Patentansprüche für folgende(n) Vertragsstaat(en): AT, BE, CH, LI, DE, DK, FR, GB, IT, LU, NL, PT, SE)

1. Anti-idiotypischer monoclonaler Antikörper, wobei dieser anti-idiotypische Antikörper gegen den für Schweine-Somatotropien spezifischen Antikörper mit der Bezeichnung PS-7.6 gebildet und vom Hybridom ATCC 10416 erhältlich ist, wobei der anti-idiotypische Antikörper die Aktivität von Schweine-Somatotropie nachahmt und das Wachstum in einem Wirbeltier anregt.

2. Zusammensetzung, umfassend:
(a) eine wirksame, das Wachstum anregende Menge des anti-idiotypischen, monoclonalen Antikörpers von Anspruch 1 und
(b) ein pharmazeutisch akzeptables Verdünnungsmittel oder einen solchen Träger.

3. Verfahren zum Anregen des Wachstums eines Wirbeltiers mit Ausnahme von Menschen, umfassend das Verabreichen einer wirksamen, das Wachstum anregenden Menge des anti-idiotypischen monoclonalen Antikörpers von Anspruch 1 an das Wirbeltier.

## Patentansprüche (Patentansprüche für folgende(n) Vertragsstaat(en): ES, GR)

1. Verfahren zum Anregen des Wachstums eines Wirbeltieres mit Ausnahme von Menschen, umfassend das Verabreichen einer wirksamen, das Wachstum anregenden Menge eines anti-idiotypischen monoclonalen Antikörpers an ein Wirbeltier, wobei der anti-idiotypische Antikörper gegen den für Schweine-Somatotropin spezifischen Antikörper mit der Bezeichnung PS-7.6 gebildet wird und erhältlich ist von Hybridom ATCC 10416, wobei der anti-idiotypische Antikörper die Aktivität von Schweine-Somatotropin nachahmt und das Wachstum im Wirbeltier anregt.

2. Verfahren zum Herstellen einer Zusammensetzung zum Anregen des Wachstums eines Wirbeltieres, umfassend das Formulieren
(a) einer wirksamen, das Wachstum anregenden Menge eines anti-idiotypischen monoclonalen Antikörpers, wobei der anti-idiotypische Antikörper gegen den für Schweine-Somatotropin spezifischen Antikörper mit der Bezeichnung PS-7.6 gebildet wird und aus Hybridom ATCC 10416 erhältlich ist, wobei der anti-idiotypische Antikörper die Aktivität von Schweine-Somatotropin nachahmt und das Wachstum in einem Wirbeltier anregt und
(b) eines pharmazeutisch akzeptablen Verdünnungsmittels oder Trägers.

3. Verfahren zum Herstellen eines anti-idiotypischen monoclonalen Antikörpers, der die Aktivität von Schweine-Somatropin nachahmt und das Wachstum in einem Wirbeltier, mit Ausnahme von Menschen, anregt, wobei das Verfahren die Stufe des Bildens eines Antikörpers gegen einen PS-7.6 bezeichneten Antikörper einschließt, der spezifisch für Schweine-Somatotropin ist und vom Hybridom ATCC 10416 erhältlich ist.

4. Verwendung eines anti-idiotypischen monoclonalen Antikörpers, der nach dem Verfahren von Anspruch 3 erhältlich ist, zum Anregen des Wachstums eines Wirbeltieres mit Ausnahme von Menschen.

5. Verwendung eines anti-idiotypischen monoclonalen Antikörpers, der gemäß dem Verfahren von Anspruch 3 erhältlich ist, zum Herstellen einer Zusammensetzung zum Anregen des Wachstums eines Wirbeltieres.

## Revendications (Revendications pour l'(les) Etat(s) contractant(s) suivant(s): AT, BE, CH, LI, DE, DK, FR, GB, IT, LU, NL, PT, SE)

1. Anticorps monoclonal anti-idiotypique, ledit anticorps anti-idiotypique étant dirigé contre l'anticorps désigné par PS-7,6 spécifique de la somatotropine porcine, et pouvant être obtenu à partir de l'hybridome ATCC 10416, ledit anticorps anti-idiotypique imitant l'activité de la somatotropine porcine et stimulant la croissance chez un vertébré.

2. Composition comprenant:
a) une quantité efficace stimulant la croissance de l'anticorps monoclonal anti-idiotypique de la revendication 1; et
b) un diluant ou un véhicule pharmaceutiquement acceptable.

3. Procédé de stimulation de la croissance d'un vertébré, à l'exception des humains, qui comprend l'administration au vertébré d'une quantité efficace stimulant la croissance de l'anticorps monoclonal anti-idiotypique de la revendication 1.

## Revendications (Revendications pour l'(les) Etat(s) contractant(s) suivant(s): ES, GR)

1. Procédé de stimulation de la croissance d'un vertébré à l'exception des humains qui comprend l'administration à un vertébré d'une quantité efficace stimulant la croissance d'un anticorps monoclonal anti-idiotypique , ledit anticorps anti-idiotypique étant dirigé contre l'anticorps désigné par PS-7,6, spécifique de la somatotropine porcine, et pouvant être obtenu à partir de l'hybridome ATCC 10416, ledit anticorps anti-idiotypique imitant l'activité de la somatotropine porcine et stimulant la croissance chez un vertébré.

2. Procédé de fabrication d'une composition destinée à stimuler la croissance d'un vertébré, comprenant la formulation :
a) d'une quantité efficace stimulant la croissance de l'anticorps monoclonal anti-idiotypique, ledit anticorpsanti-idiotypique étant dirigé contre l'anticorps désigné par PS-7,6, spécifique de la somatotropine porcine, et pouvant être obtenu à partir de l'hybridome ATCC 10416, ledit anticorps anti-idiotypique imitant l'activité de la somatotropine porcine et stimulant la croissance chez un vertébré; et
b) d'un diluant ou un véhicule pharmaceutiquement acceptable.

3. Procédé de fabrication d'un anticorps monoclonal anti-idiotypique imitant l'activité de la somatotropine porcine et stimulant la croissance d'un vertébré à l'exception des humains, ledit procédé comprenant l'étape consistant à produire un anticorps dirigé contre un anticorps désigné par PS-7,6, spécifique de la somatotropine porcine, et pouvant être obtenu à partir de l'hybridome ATCC 10416.

4. Utilisation d'un anticorps monoclonal anti-idiotypique pouvant être obtenu selon le procédé de la revendication 3 pour stimuler la croissance d'un animal, à l'exception des humains.

5. Utilisation d'un anticorps monoclonal anti-idiotypique pouvant être obtenu selon le procédé de la revendication 3 pour fabriquer une composition destinée à stimuler la croissance d'un vertébré.
